# FASCICULE DE BREVET EUROPEEN

(11) **EP 1 539 195 B1**
(45) Date de publication et mention de la délivrance du brevet: **09.01.2013**
(21) Numéro de dépôt: 03773775.6
(22) Date de dépôt: 12.09.2003
(51) Int. Cl.: A61K 31/716, A23L 1/09, A23L 1/308, C12P 19/18, C07H 3/06

(54) **PREBIOTIQUES DE GLUCOOLIGOSACCHARIDES POUR LA PREVENTION DU DIABETE DE TYPE II**
GLUKOOLIGOSACCHARID PREBIOTIKEN ZUR VORBEUGUNG VOM DIABETES TYP II
GLUCOOLIGOSACCHARIDE PREBIOTICS FOR THE PREVENTION OF DIABETES

(30) Priorité: 13.09.2002 FR 0211389
(43) Date de publication de la demande: 15.06.2005
(73) Titulaire: INSTITUT NATIONAL DE LA RECHERCHE AGRONOMIQUE (INRA), 75338 Paris Cédex 07 (FR)
(72) Inventeur: MONSAN, Pierre, F-31700 Mondonville (FR); VALET, Philippe, F-31000 Toulouse (FR); REMAUD-SIMEON, Magali, F-31520 Ramonville-Saint-Agne (FR); SAULNIER-BLACHE, Jean-Sébastien, F-31130 Balma (FR); BURCELIN, Rémy, F-31450 ESCALQUENS (FR)
(74) Mandataire: Grosset-Fournier, Chantal Catherine
(86) Numéro de dépôt international: PCT/FR2003/002705
(87) Numéro de publication internationale: WO 2004/024167

(56) Documents cités:
- EP-A- 0 325 872
- EP-A- 1 156 057
- EP-A- 1 332 759
- DE-A- 19 961 182
- US-A- 5 817 634
- US-A- 5 981 510
- ROBERFROID M B: "FUNCTIONAL FOODS: CONCEPTS AND APPLICATION TO INULIN AND OLIGOFRUCTOSE" BRITISH JOURNAL OF NUTRITION, CAMBRIDGE, GB, vol. 87, no. SUPPL 2, mai 2002 (2002-05), pages S139-S143, XP009008910
- CHUNG C ET AL: 'Glucooligosaccharides from Leuconostoc mesenteroides B-742, a potential prebiotic' ABSTRACTS OF THE GENERAL MEETING OF THE AMERICAN SOCIETY FOR MICROBIOLOGY vol. 101, 2001, & 101ST GENERAL MEETING OF THE AMERICAN SOCIETY FOR MICROBIOLOGY; ORLANDO, FL, USA; MAY 20-24, 2001, page 552, XP009027712 ISSN: 1060-2011
- BOUCHER J ET AL: 'Effect of non-digestible gluco-oligosaccharides on glucose sensitivity in high fat diet fed mice.' JOURNAL OF PHYSIOLOGY AND BIOCHEMISTRY. SEP 2003 vol. 59, no. 3, Septembre 2003, pages 169 - 173, XP009027711 ISSN: 1138-7548
- ROBERFROID M B: 'Prebiotics and probiotics: are they functional foods?' THE AMERICAN JOURNAL OF CLINICAL NUTRITION. JUN 2000 vol. 71, no. 6 SUPPL, Juin 2000, pages 1682S - 7S ; DISC, XP001180260 ISSN: 0002-9165

## Description

La présente invention a pour objet l'utilisation de prébiotiques afin de traiter et de prévenir les syndromes hyperglycémiques.

Les prébiotiques sont des composés alimentaires non-digestibles dégradés par les micro-organismes de la flore intestinale et dont la dégradation est responsable d'effets bénéfiques pour la santé de l'hôte. Les effets bénéfiques sur la santé sont dus à une stimulation sélective de la croissance et/ou de l'activité biologique de certains micro-organismes de la flore intestinale, notamment les bifodobactéries et les bactéries lactiques de la flore colique.

La demande de brevet européen EP 0,325,872 décrit un procédé de préparation de gluco-saccharides par synthèse enzymatique impliquant la glucosyltransférase. Il mentionne uniquement l'effet prébiotique des gluco-oligosaccharides sans préciser leur effets bénéfiques sur la santé.

Le document Chuang et al.(« Glucooligosaccharides from Leuconostoc mesenteroides B-742, a Potential Prebiotic », vol.101, 2001, page 552, ASM 101st General Meeting) divulgue l'utilisation de gluco-oligosaccharides et de fructo-oligosaccharides comme probiotiques.

Le brevet européen 0,382,355 décrit l'utilisation de gluco-oligosaccharides, de fructo-oligosaccharides et d'isomalto-oligasaccharides dans la prévention des maladies gériatriques telles que le diabète de type II.

La demande internationale WO 00/70964 mentionne l'effet des gluco-oligosaccharides, des fructo-oligosaccharides et des xylo-oligosaccharides sur la sensibilité à l'insuline.

Les effets des prébiotiques sont principalement dus à la stimulation de la croissance des bifidobactéries (effet bifidogène). La stimulation de cette croissance permet d'abaisser le pH du colon, d'augmenter la production d'acides gras à chaîne courte, notamment le butyrate et le propionate, d'empêcher l'installation de micro-organismes pathogènes (effet barrière), d'augmenter la métabolisation de composés aminés potentiellement cancérigènes et la production de vitamine B.

L'utilisation des prébiotiques permet également de stimuler le système immunitaire grâce à la production d'acide lipothéichoïques par les bactéries, l'interaction de ces bactéries avec les plaques de Peyer et la stimulation de la circulation lymphocytaire périphérique.

Les prébiotiques favorisent également l'absorption digestive de minéraux, en particulier le calcium et le magnésium, ce qui permet d'envisager leur application potentielle dans le cadre du traitement de l'ostéoporose.

En l'état actuel les seuls prébiotiques clairement définis sont des sucres classés parmi les fibres alimentaires: les oligosaccharides (également appelés oligosides) non digestibles.

Les oligosaccharides sont des polymères d'oses de faible degré de polymérisation. Le nombre d'unité osidique est typiquement de 2 à 12 unités avec une moyenne à environ 3-5 unités. Les oses entrant dans la formation des oligosaccharides sont variés, on trouve notamment des hexoses comme le glucose, le galactose, et le fructose, mais également des pentoses comme le xylose.

Les oligosaccharides peuvent être constitués d'un seul type d'oses (homo-oligosides) ou d'un mélange (hétéro-oligosides). Les types de liaisons entre les unités osidiques sont multiples : α(1→2), α(1→4), α(1→6), β(1→4), β(2→1).

Les oligosaccharides peuvent être issus: de la dégradation de polymères naturels tels que l'amidon ou l'inuline, d'extractions directes à partir de substances naturelles, comme le soja, ou de synthèses chimiques ou enzymatiques.

Les gluco-oligosaccharides (gluco-oligosides) (GOS) constituent une classe importante d'oligosaccharides. Il s'agit de l'ensemble des oligosaccharides constitués de l'enchaînement de glucoses suivant la formule générale (O-α-D-gucopyranosyl)ₙ ou n est un nombre entier de 2 à 10. Il est possible de différencier:
- les malto-oligosaccharides, issus de l'hydrolyse de l'amidon et répondant à la formule générale : [O-α-D-gucopyranosyl-(1→4)]ₙ où n vaut de 2 à 10,
- les isomalto-oligosaccharides, également issus de la transformation enzymatique d'hydrolysat d'amidon et répondant à la formule générale [O-α-D-gucopyranosyl-(1 → 6)]ₙ où n est un nombre entier de 2 à 10,
- les oligodextranes comportant des résidus glucose (Glu) reliés par des liaisons de type α(1 →6) et comportant au moins une liaison de type α(1→2). Ce type de polymère est obtenu par synthèse enzymatique, en particulier à partir de maltose et de saccharose en présence d'une glucosyltransférase. La formule générale de ce type de composé est [Glu α(1→2)] [Glu α(1→6)]ₙ [Glu α(1→4)]Glu ou n est un nombre entier de 1 à 10 et la position de la liaison α(1→2) se situe soit à l'extrémité non réductrice, soit se situe en ramification sur l'avant dernier glucose de la chaîne. La présence de cette liaison α(1→2) confère des propriétés particulières à cet oligosaccharide ; en effet le système digestif humain ne possède pas l'équipement enzymatique nécessaire à l'hydrolyse de ce type de liaison. La stabilité des oligodextranes possédant ce type de liaison leur permet donc aisément de transiter, sans être digérés, jusqu'au gros intestin où il peuvent servir de substrat spécifique à la flore microbienne colique. Ces polymères possèdent donc des caractéristiques les rattachant aux prébiotiques.

A titre d'illustration, des GOS sont plus particulièrement décrits dans la demande de brevet européen déposée le 2 août 1989, et publiée sous le numéro 0 325 872.

Quelques études sur les interactions des oligosaccharides avec le métabolisme glucidique ont été réalisées chez le diabétique non-insulino-dépendant et chez le sujet sain, et ont mis en évidence un possible impact sur la glucorégulation. Cependant, l'effet des oligosaccharides prébiotiques sur les troubles du métabolisme glucidique et leur installation chez le sujet présentant une surcharge pondérale ou obèse ne sont pas connus.

Parmi les syndromes hyperglycémiques, le diabète de type II associé à une obésité, diabète sucré non-insulino-dépendant, constitue un problème de santé publique croissant dans les pays industrialisés, en grande partie à cause de la prévalence accrue de l'obésité, elle-même due à une alimentation trop riche.

La présente invention découle de la mise en évidence par les Inventeurs du fait que des souris soumises à un régime alimentaire hyper-lipidique et ayant reçu l'administration de prébiotiques ne présentent pas le phénomène d'intolérance au glucose que l'on détecte pour le groupe de souris témoins non traitées aux prébiotiques, et donc que ces souris traitées aux prébiotiques se comportent comme des individus sains et ne développent pas de diabète de type II, à la différence du groupe non traité.

La présente invention découle également de la mise en évidence par les Inventeurs que la glycémie de souris atteintes de diabète de type II pouvait être abaissée grâce à un traitement à base de prébiotiques.

Ainsi l'invention a principalement pour but de fournir des aliments, alicaments, nutraceutiques, ou médicaments, destinés au traitement et/ou à la prévention des syndromes hyperglycémiques et notamment au traitement du diabète de type II et/ou à la prévention de l'installation du diabète de type II chez les sujets à risque.

A ce titre, la présente invention a pour objet l'utilisation de prébiotiques pour la préparation de compositions alimentaires ou pharmaceutiques destinées à la prévention des syndromes hyperglycémiques et à la prévention de l'apparition d'un diabète de type II chez les sujets présentant une prédisposition à développer ce type de diabète, à savoir chez les sujets présentant des signes cliniques prédictifs de ce diabète, tels qu'une diminution de la tolérance au glucose, ou de la sensibilité à l'insuline, notamment chez les sujets présentant une prédisposition héréditaire à développer ce type de diabète, ou liée à leurs habitudes alimentaires, lesdits sujets étant atteints d'obésité, ou susceptibles de développer une obésité,
lesdits prébiotiques étant choisis parmi les compositions d'oligosaccharides non digestibles comprenant des enchaînements d'oses identiques ou différents, et dont le degré de polymérisation varie entre 2 et 10, et de préférence entre 3 et 8,
lesdits oligosaccharides étant choisis parmi:
- les gluco-oligosaccharides (GOS), à savoir des polymères de glucose de formule générale [O-α-D-glucopyranosyl]ₙ où n est un nombre entier de 2 à 10, et de préférence de 3 à 8, tels que les polymères de formule générale [O-α-D-glucopyranosyl-(1→2)][O-α-D-glucopyranosyl-(1→6)ₙ[O-α-D-glucopyranosyl-(1→4)]O-D-glucopyranose où n est un nombre entier de 1 à 10, et la position de la liaison α(1→2) se situe soit à l'extrémité non réductrice, soit se situe en ramification sur l'avant dernier glucose de la chaîne, ou les polymères de type malto-oligosaccharides de formule générale [O-α-D-glucopyranosyl-(1→4)]ₙ où n est un nombre entier de 2 à 10, ou les isomalto-oligosaccharides de formule générale [O-α-D-glucopyranosyl-(1→6)]ₙ où n est un nombre entier de 2 à 10,
la composition desdits gluco-oligosaccharides (GOS) étant la suivante (teneur en matière sèche) :
- fructose : moins de 1%,
- glucose : moins de 4%,
- disaccharides (maltose, leucrose, sacharose) : de 9 à 11%,
- trisaccharides (panose, maltotriose) : de 9 à 11%,
- GOS de degré de polymérisation 4 : de 5 à 7%,
- GOS* de degré de polymérisation 4 : de 8 à 10%.
- GOS de degré de polymérisation 5 : de 18 à 22%,
- GOS de degré de polymérisation supérieur à 5 : de 36 à 44%,
chaque GOS comportant une liaison glycosidique α(1→2) se situant soit à l'extrémité non réductrice, soit se situant en ramification sur l'avant dernier glucose de la chaîne, excepté le GOS* marqué par un astérisque qui n'en contient pas.

Par « prébiolique » on désigne des composés alimentaires non-digestibles dont l'effet bénéfique sur la santé est dû à une stimulation sélective de la croissance et/ou de l'activité biologique de certaine(s) bactérie(s) de la flore intestinale.

On désigne par « syndrome hyperglycémique » toute pathologie caractérisée par une glycémie anormalement élevée, c'est-à-dire toute pathologie caractérisée par une glycémie à jeun supérieure à environ 7,6 mmol/l. Les syndromes hyperglycémiques comprennent notamment les diabètes de type I et II.

L'invention décrit que les oses des compositions oligosaccharidiques susmentionnées peuvent être choisis parmi le glucose, le fructose, le galactose, le xylose, le mannose, le rhamnose et le fucose.

L'invention décrit que les prébiotiques dans l'utilisation susmentionnée peuvent être choisis parmi:- les fructo-oligosaccharides (FOS) de formule générale O-α-D-glucopyranosyl-(1→2)-[O-β-D-fructofuranosyl-(1→2)]ₙ ou [O-β-D-fructofuranosyl-(1→2)]m où n est un nombre entier de 2 à 9, et m est un nombre entier de 1 à 9,
- les galacto-oligosaccharides de formule générale O-α-D-glucopyranosyl- (1→4)-[O-β-D-galactopyranosyl-(1→6)]ₙ où n est un nombre entier de 2 à 5,
- les xylo-oligosaccharides de formule générale [O-β-xylofuranosyl-(1→4)]ₙ où n est un nombre entier de 2 à 9,
- les oligosaccharides de soja tels que le raffinose de formule O-α-D-galactopyranosyl-(1→6)-O-α-D-glucopyranosyl-(1→2)-O-β-D-fructofuranoside et le stachyose de formule [O-α-D-galactopyranosyl-(1→6)]₂-O-α-D-glucopyranosyl-(1→2)-O-β-D-fructofuranoside,
- le lactulose de formule O-β-D-galactopyranosyl-(1→4)-O-β-D-fructofuranose.
- le lactosaccharose de formule O-β-D-galactopyranosyl-(1→4)-O-α-D-glucopyranosyl-(1→2)-O-β-D-fructofuranoside. L'invention a décrit que des prébiotiques dans l'utilisation susmentionnée peuvent être choisis parmi les gluco-oligosaccharides (GOS) tels que définis ci-dessus, et plus particulièrement des GOS décrits dans la demande de brevet européen déposée le 2 août 1989, et publiée sous le numéro 0 325 872.

Selon un mode de réalisation particulier, les GOS de la composition ci-dessus correspondent à la formule générale [Glu α(1→2)] [Glu α(1→6)]ₙ [Glu α(1→4)]Glu (n ≥ 1), la liaison glycosidique (α(1→2) se situant soit à l'extrémité non réductrice, soit se situant en ramification sur l'avant dernier glucose de la chaîne, excepté pour le GOS* (marqué par un astérisque) qui correspond à la formule générale [Glu α(1→6)]ₙ [Gluα(1→4)]Glu (n = 2).

L'invention concerne également l'utilisation susmentionnée de prébiotiques tels que définis ci-dessus pour la préparation de compositions alimentaires ou pharmaceutiques destinées à la prévention des syndromes hyperglycémiques et à la prévention de l'apparition d'un diabète, de type II chez les sujets présentant une prédisposition à développer ce type de diabète, à raison d'environ 10 à 30 g/jour, jusqu'à environ 100 g/jour dans le cas de l'utilisation de GOS.

L'invention a également pour objet les compositions alimentaires, additifs nutritionnels, alicaments, ou nutraceutiques, comprenant un ou plusieurs prébiotiques choisis parmi les compositions d'oligosaccharides non digestibles comprenant des enchaînements d'oses identiques ou différents, et dont le degré de polymérisation varie entre 2 et 10, et de préférence entre 3 et 8, pour son utilisation dans l'alimentation des sujet risquant de développer un syndrome hyperglycémique, dans le cadre de la prévention des syndromes hyperglycémiques, et dans l'alimentation des sujets atteints d'obésité, ou susceptibles de développer une obésité, et présentant une prédisposition à développer un diabète de type II, à savoir chez les sujets présentant des signes cliniques prédictifs de ce diabète, tels qu'une diminution de la tolérance au glucose, ou de la sensibilité à l'insuline, notamment chez les sujets présentant une prédisposition héréditaire à développer ce type de diabète, ou liée à leurs habitudes alimentaires, dans le cadre de la prévention de l'apparition d'un diabète de type II chez ces sujets,
lesdits oligosaccharides étant choisis parmi :
- les gluco-oligosaccharides (GOS), à savoir des polymères de glucose de formule générale [O-α-D-glucopyranosyl]ₙ où n est un nombre entier de 2 à 10, et de préférence de 3 à 8, tels que les polymères de formule générale [O-α-D-glucopyranosyl-(1→2)][O-α-D-glucopyranosyl-(1→6)]ₙ[O-α-D-glucopyranosyl-(1→4)jO-D-glucopyranose où n est un nombre entier de 1 à 10, et la position de la liaison α(1→2) se situe soit à l'extrémité non réductrice, soit se situe en ramification sur l'avant dernier glucose de la chaîne, ou les polymères de type malto-oligosaccharides de formule générale [O-α-D-glucopyranosyl-(1→4)]ₙ où n est un nombre entier de 2 à 10, ou les isomalto-oligosaccharides de formule générale [O-α-D-glucopyranosyl-(1→6)]ₙ où n est un nombre entier de 2 à 10,
la composition desdits gluco-oligosaccharides (GOS) étant la suivante (teneur en matière sèche) :
- fructose : moins de 1%,
- glucose : moins de 4%,
- disaccharides (maltose, leucrose, sacharose) : de 9 à 11%,
- trisaccharides (panose, maltotriose) : de 9 à 11%,
- GOS de degré de polymérisation 4 : de 5 à 7%,
- GOS* de degré de polymérisation 4 : de 8 à 10%,
- GOS de degré de polymérisation 5 : de 18 à 22%,
- GOS de degré de polymérisation supérieur à 5 : de 36 à 44%,
chaque GOS comportant une liaison glycosidique α(1→2) se situant soit à l'extrémité non réductrice, soit se situant en ramification sur l'avant dernier glucose de la chaîne, excepté le GOS* marqué par un astérisque qui n'en contient pas. L'invention décrit aussi que les oses des compositions oligosaccharidiques dans toute composition alimentaire, additif nutritionnel, alicament ou nutraceutique, tels que définis ci-dessus, peuvent être choisis parmi le glucose, le fructose, le galactose, le xylose, le mannose, le rhamnose et le fucose.

L'invention décrit aussi que les prébiotiques dans toute composition alimentaire, additif nutritionnel, alicament ou nutraceutique, tels que définis ci-dessus, peuvent être choisis parmi :- les fructo-oligosaccharides (FOS) de formule générale O-α-D-glucopyranosyl-(1→2)-(O-β-D-fructofuranosyl-(1→2)ₙ ou [O-β-D-fructofuranosyl-(1→2)]ₘoù n est un nombre entier de 2 à 9, et m est un nombre entier de 1 à 9,
- les galacto-oligosaccharides de formule générale O-α-D-glucopyranosyl-(1→4)-[O-β-D-galactopyranosyl-(1→6)]ₙ où n est un nombre entier de 2 à 5,
- les xylo-oligosaccharides de formule générale [O-β-xylofuranosyl-(1 →4)]ₙ où n est un nombre entier de 2 à 9,
- les oligosaccharides de soja tels que le raffinose de formule O-α-D-galactopyranosyl-(1→6)-O-α-D-glucopyranosyl-(1→2)-O-β-D-fructofuranoside et le stachyose de formule [O-α-D-galactopyranosyl-(1→6)]₂-O-α-D-glucopyranosyl-(1→2)-O-β-D-fructofuranoside,
- le lactulose de formule O-β-D-galactopyranosyl-(1→4)-O-β-D-fructofuranose,
- le lactosaccharose de formule O-β-D-galactopyranosyl-(1→4)-O-α-D-glucopyranosyl-(1 →2)-O-β-D-fructofuranoside.

L'invention décrit également que les prébiotiques dans les compositions alimentaires, additifs nutritionnels alicaments ou nutraceutiques, tels que définis ci-dessus, peuvent être choisis parmi les gluco-oligosaccharides (GOS) susmentionnés.

Selon un mode de réalisation particulier les GOS de la composition ci-dessus correspondent à la formule générale [Glu α(1→2)] [Glu α(1→6)]ₙ [Glu α(1→4)]Glu (n ≥ 1), la liaison glycosidique α(1→2) se situant soit à l'extrémité non réductrice, soit se situant en ramification sur l'avant dernier glucose de la chaîne, excepté pour le GOS* (marqué par un astérisque) qui correspond à la formule générale [Glu α(1→6)]ₙ [Gluα(1→4)]Glu (n = 2).

L'invention concerne également toute composition pharmaceutique caractérisée en ce qu'elle comprend un ou plusieurs prébiotiques en association avec un véhicule pharmaceutiquement acceptable.

L'invention concerne plus particulièrement toute composition pharmaceutique telle que définie ci-dessus, caractérisée en ce qu'elle comprend un ou plusieurs prébiotiques choisis parmi les compositions d'oligosaccharides non digestibles comprenant des enchaînements d'oses identiques ou différents, et dont le degré de polymérisation varie entre 2 et 10, et de préférence entre 3 et 8,
lesdits prébiotiques étant choisis parmi :
- les gluco-oligosaccharides (GOS), à savoir des polymères de glucose de formule générale [O-α-D-glucopyranosyl]ₙ où n est un nombre entier de 2 à 10, et de préférence de 3 à 8, tels que les polymères de formule générale [O-α-D-glucopyranosyl-(1→2)][O-α-D-glucopyranosyl-(1→6)]ₙ[O-α-D-glucopyranosyl-(1→4)]O-D-glucopyranose où n est un nombre entier de 1 à 10, et la position de la liaison α(1→2) se situe soit à l'extrémité non réductrice, soit se situe en ramification sur l'avant dernier glucose de la chaîne, ou les polymères de type malto-oligosaccharides de formule générale [O-α-D-glucopyranosyl-(1→4)]ₙ où n est un nombre entier de 2 à 10, ou les isomalto-oligosaccharides de formule générale [O-α-D-glucopyranosyl-(1→6)]ₙ où n est un nombre entier de 2 à 10,
la composition desdits gluco-oligosaccharides (GOS) étant la suivante (teneur en matière sèche) :
- fructose : moins de 1%,
- glucose : moins de 4%,
- disaccharides (maltose, leucrose, sacharose) : de 9 à 11%,
- trisaccharides (panose, maltotriose) : de 9 à 11 %,
- GOS de degré de polymérisation 4 : de 5 à 7%,
- GOS* de degré de polymérisation 4 : de 8 à 10%,
- GOS de degré de polymérisation 5 : de 18 à 22%,
- GOS de degré de polymérisation supérieur à 5 : de 36 à 44%,
chaque GOS comportant une liaison glycosidique α(1→2) se situant soit à l'extrémité non réductrice, soit se situant en ramification sur l'avant dernier glucose de la chaîne, excepté le GOS* marqué par un astérisque qui n'en contient pas.

L'invention décrit que les oses des compositions oligosaccharidiques dans toute composition pharmaceutique telle que définie ci-dessus peuvent être choisis parmi le glucose, le fructose, le galactose, le xylose, le mannose, le rhamnose et le fucose.L'invention décrit que les prébiotiques dans toute composition pharmaceutique telle que définie ci-dessus, peuvent être choisis parmi :
- les fructo-oligosaccharides (FOS) de formule générale O-α-D-glucopyranosyl-(1→2)-[O-β-D-fructofuranosyl-(1→2)]ₙ ou [O-β-D-fructofuranosyl-(1→2)]ₘ où n est un nombre entier de 2 à 9, et m est un nombre entier de 1 à 9,
- les galacto-oligosaccharides de formule générale O-α-D-glucopyranosyl-(1→4)-[O-β-D-galactopyranosyl-(1→6)]ₙ où n est un nombre entier de 2 à 5,
- les xylo-oligosaccharides de formule générale [O-β-xylofuranosyl-(1→4)]ₙ où n est un nombre entier de 2 à 9,
- les oligosaccharides de soja tels que le raffinose de formule O-α-D-galactopyranosyl-(1→6)-O-α-D-glucopyranosyl-(1→2)-O-β-D-fructofuranoside et le stachyose de formule [O-α-D-galactopyranosyl-(1→6)]₂-O-α-D-glucopyranosyl-(1→2)-O-β-D-fructofuranoside,
- le lactulose de formule O-β-D-galactopyranosyl-(1→4)-O-β-D-fructofuranose,
- le lactosaccharose de formule O-β-D-galactopyranosyl-(1→4)-O-α-D-glucopyranosyl-(1→2)-O-β-D-fructofuranoside.

L'invention décrit aussi que les prébiotiques dans toute composition pharmaceutique telle que définie ci-dessus, peuvent être choisis parmi les gluco-oligosaccharides (GOS) susmentionnés.

Selon un mode de réalisation particulier les GOS de la composition ci-dessus correspondent à la formule générale (Glu α(1→2)] [Glu α(1→6)]ₙ [Glu α(1→4)]Glu (n ≥ 1), la liaison glycosidique α(1→2) se situant soit à l'extrémité non réductrice, soit se situant en ramification sur l'avant dernier glucose de là chaîne, excepté pour le GOS* (marqué par un astérisque) qui correspond à la formule générale [Glu α(1→6)]ₙ [Gluα(1→4)]Glu (n = 2).

Avantageusement les compositions pharmaceutiques susmentionnées se présentent sous une forme administrable par voie orale.

De préférence, les compositions pharmaceutiques susmentionnées sont destinées à être administrées à raison d'environ 10 à 30 g/jour, jusqu'à environ 100 g/jour dans le cas de l'utilisation de GOS.

L'invention sera davantage illustrée à l'aide de la description détaillée qui suit, du traitement et de la prévention de l'installation d'un diabète de type II chez des souris soumises à un régime hyper-lipidique et traitées par des prébiotiques tels que définis ci-dessus.

### DESCRIPTION DES FIGURES

### Figure 1

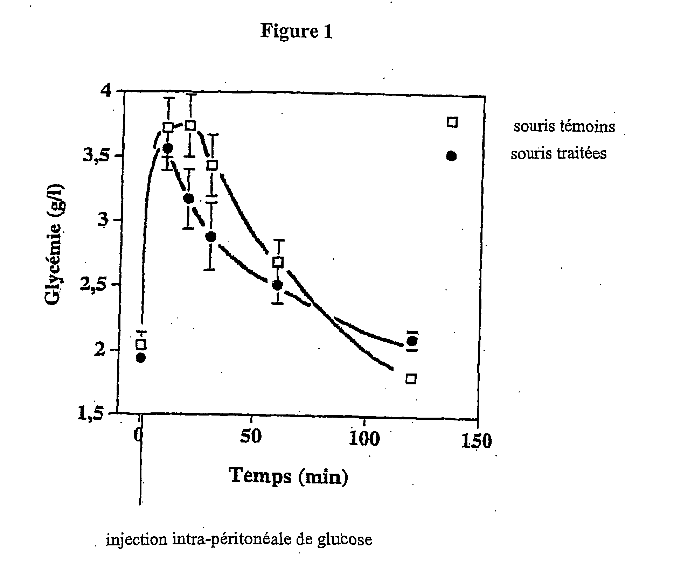

Le graphe de la figure 1 représente l'évolution de la glycémie (en ordonnée, g/l), en fonction du temps (en abscisse, min.), suite à l'injection intra-péritonéale de glucose (1 g/kg) à des souris soumises à un régime hyperlipidique. Les valeurs moyennes de la glycémie de 6 souris témoins sont représentées par des carrés blancs, les valeurs correspondant à 6 souris dont le régime alimentaire était supplémenté en GOS sont représentées par des cercles noirs.

### Figure 2

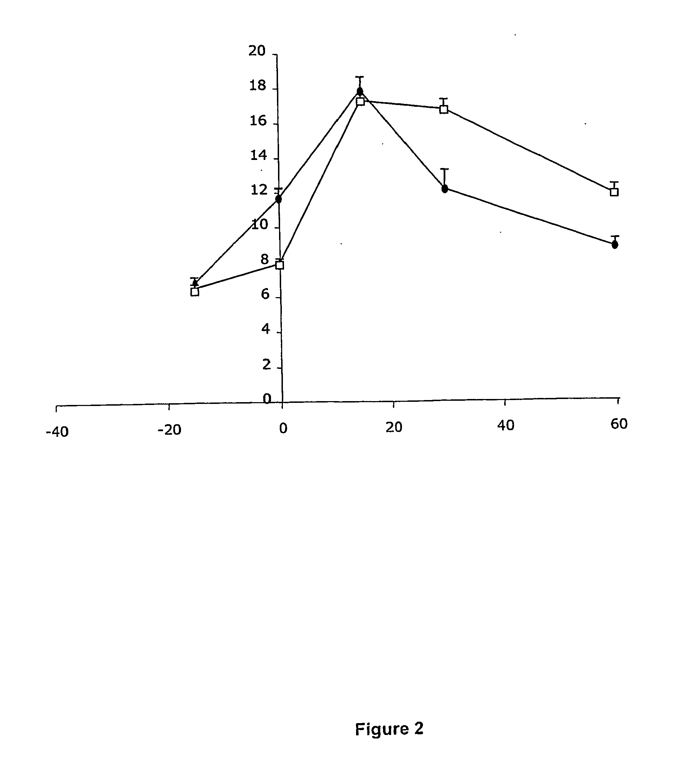

Le graphe de la figure 2 représente l'évolution de la glycémie (en ordonnée, mM), en fonction du temps (en abscisse, min.), suite à l'administration orale de glucose (1 g/kg, temps 0 min.) à des souris atteinte de diabète de type II. Les valeurs moyennes de la glycémie de 15 souris témoins sont représentées par des carrés blancs, les valeurs correspondant à 15 souris auxquelles ont été administrés des GOS (temps -15 min.) sont représentées par des cercles noirs.

### EXEMPLES

### Exemple 1

### Prévention de l'installation du diabète de type II par traitement chronique aux GOS

### I. Protocole experimental

### 1. Installation de l'obésité

12 souris femelles C57B6 âgées de 8 semaines ont été soumises à un régime hyper-lipidique durant 20 semaines.

La ration alimentaire, fournie *ad libitum,* comprenait (en %) :

| | |
|---|---|
| Amidon de maïs : | 20 |
| Saindoux : | 20 |
| Caséine: | 20 |
| Maltodextrine : | 2 |
| Saccharose: | 22 |
| Cellulose : | 5 |
| Vitamines : | 1 |
| CM205b : | 7 |
| Huile de soja : | 3 |

### 2. Traitement chronique aux gluco-oligosaccharides (GOS)

Les souris ont été séparées en 4 groupes de 3 souris comprenant 2 groupes de souris traitées et 2 groupes de souris témoins.

Durant toute la durée du régime hyper-lipidique les souris traitées ont reçu dans leur eau de boisson 1,5 g/kg/j d'une composition de gluco-oligosaccharides, soit 45 mg/j/souris, ce qui correspond à l'ajout de 1g de produit par semaine et par cage dans 100 mL d'eau.

La prise hydrique et la prise alimentaire ont été vérifiées et la prise de poids a fait l'objet d'une mesure hebdomadaire:

La composition de gluco-oligosaccharide (BioEurope-Solabia) fournie est la suivante (teneur en % de matière sèche) :

| **Sucre** | **Teneur en matière sèche (en %)** |
|---|---|
| fructose | moins de 1 |
| glucose | moins de 4 |
| disaccharides (maltose, leucrose, sacharose) | de 9 à 11 |
| trisaccharides (panose, maltotriose) | 9 à 11 |
| GOS (d.p. 4) | 5 à 7 |
| GOS* (d.p. 4) | 8 à 10 |
| GOS (d.p. 5) | 18 à 22 |
| GOS (d.p. >5) | 36 à 44 |

| | |
|---|---|
| *Les GOS utilisés correspondent à la formule générale [Glu α(1 → 2)] [Glu α(1 →6)]ₙ [Glu α(1 →4)]Glu, la liaison glycosidique α(1 →2) se situant soit à l'extrémité non réductrice, soit se situant en ramification sur l'avant dernier glucose de la chaîne, excepté pour le GOS* (marqué par un astérisque) qui ne contient pas de liaison α(1 →2).* | |

### 3. Mesures à 20 semaines

Au terme des 20 semaines diverses mesures ont été réalisées :
- les urines ont été recueillies sur 24h afin de mesurer la glucosurie à l'aide d'un appareil *diaburtest 5000* (Roche)
- la glycémie à jeun a été mesurée à l'aide d'un appareil *Glucotrend plus* (Roche)
- les souris ont été soumises à un test de tolérance au glucose : injection intra-péritonéale de glucose à raison de 1 g/kg et suivi de la glycémie sur 120 min. à l'aide d'un appareil *Glucotrend plus* (Roche) (voir **Figure 1****)**.
- après sacrifice des souris les principaux tissus impliqués dans la glycorégulation (sang, foie, tissu adipeux, muscle, rein) ont été prélevés.

### II. Résultats

Les contrôles réalisés montrent que la surcharge pondérale des animaux témoins et traités sont semblables et les dépôts graisseux équivalents. De plus la glucosurie et la glycémie a jeun sont normales, ce qui indique que l'installation du diabète lié à l'obésité n'est pas réalisée.

Le test de tolérance au glucose **(****Figure 1****)** indique en revanche que les souris témoins présentent une résistance au glucose alors que ce phénomène est significativement corrigé chez les souris traitées.

L'intolérance au glucose est considérée comme un des premiers symptômes de l'installation du diabète de type II. Il apparaît donc que le traitement aux gluco-olgosaccharides est suffisant pour éviter l'installation de ce diabète dans un modèle murin d'obésité.

### Exemple 2

### Traitement du diabète de type II par Traitement aigu aux GOS

### 1. Protocole expérimental

### 1. Installation du diabète de type II

30 souris mâles C57B6 âgées de 12 semaines ont été soumises à un régime hyper-lipidique durant 8 semaines.

La ration alimentaire, fournie *ad libitum,* comprenait (en %) :

| | |
|---|---|
| Caséine : | 37 |
| Cellulose : | 10 |
| Vitamines : | 1 |
| Huile de maïs : | 14,5 |
| Saindoux: | 35 |
| Sels minéraux : | 2,5 |

L'installation du diabète de type II a été vérifiée par la mesure de la glycémie à jeun ainsi que par un test de tolérance au glucose, selon les procédures décrite dans l'Exemple 1.

### 2. Traitement aigu aux gluco-oligosaccharides (GOS)

Les souris diabétiques ont été séparée en 2 groupes de 15 souris chacune. Le premier groupe a été traité par une administration orale de 1,5 g/kg d'une composition de GOS identique à celle utilisée dans l'exemple 1, le deuxième groupe n'a pas reçu de GOS. 15 minutes après, une administration orale de 1 g/kg de glucose a été effectuée pour les deux groupes.

La glycémie a été mesurée avant l'administration de GOS (-15 min.), avant celle de glucose (0 min.), puis aux temps 15, 30 et 60 min. après l'administration de glucose (voir Figure 2)

### II. Résultats

L'administration orale du mélange de GOS induit une diminution plus rapide et plus marquée de la glycémie significative 30 min. après l'administration de glucose (Figure 2). L'administration orale d'un mélange de GOS réduit l'intolérance au glucose des souris diabétiques.

Par conséquent, en plus de leur rôle dans la prévention de l'installation du diabète de type II, il apparaît que les GOS permettent d'abaisser la glycémie dans un modèle murin de diabète de type II.

## Revendications

1. Utilisation de prébiotiques pour la préparation de compositions alimentaires ou pharmaceutiques destinées à la prévention des syndromes hyperglycémiques et à la prévention de l'apparition d'un diabète de type II chez les sujets présentant une prédisposition à développer ce type de diabète, lesdits prébiotiques étant choisis parmi les compositions d'oligosaccharides non digestibles comprenant des enchaînements d'oses identiques ou différents, et dont le degré de polymérisation varie entre 2 et 10, et de préférence entre 3 et 8, lesdits oligosaccharides étant choisis parmi les gluco-oligosaccharides (GOS), à savoir des polymères de glucose de formule générale [O-α-D-glucopyranosyl]ₙ où n est un nombre entier de 2 à 10, et de préférence de 3 à 8, tels que les polymères de formule générale [O-α-D-glucopyranosyl-(1→2)][O-α-D-glucopyranosyl-(1→6)]ₙ[O-α-D-glucopyranosyl-(1→4)]O-D-glucopyranose où n est un nombre entier de 1 à 10, et la position de la liaison α(1 → 2) se situe soit à l'extrémité non réductrice, soit se situe en ramification sur l'avant dernier glucose de la chaîne, ou les polymères de type malto-oligosaccharides de formule générale [O-α-D-glucopyranosyl-(1→4)]ₙ où n est un nombre entier de 2 à 10, ou les isomalto-oligosaccharides de formule générale [O-α-D-glucopyranosyl-(1→6)]ₙ où n est un nombre entier de 2 à 10,
la teneur en matière sèche de la composition desdits gluco-oligosaccharides (GOS) étant la suivante:
- fructose : moins de 1%,
- glucose : moins de 4%,
- disaccharides (maltose, leucrose, sacharose) : de 9 à 11%,
- trisaccharides (panose, maltotriose) : de 9 à 11%,
- GOS de degré de polymérisation 4 : de 5 à 7%,
- GOS* de degré de polymérisation 4 : de 8 à 10%,
- GOS de degré de polymérisation 5 : de 18 à 22%,
- GOS de degré de polymérisation supérieur à 5 : de 36 à 44%,
chaque GOS comportant une liaison glycosidique α(1→2) à son extrémité non-réductrice ou portée par l'avant dernier glucose, excepté le GOS* marqué par un astérisque qui n'en contient pas.

2. Utilisation de prébiotiques selon la revendication 1 pour la préparation de compositions alimentaires ou pharmaceutiques destinées à la prévention des syndromes hyperglycémiques et à la prévention de l'apparition d'un diabète de type II chez les sujets présentant une prédisposition à développer ce type de diabète, à raison d'environ 10 à 30 g/jour, jusqu'à environ 100 g/jour.

3. Composition alimentaire, additif nutritionnel, alicament, ou nutraceutique, comprenant un ou plusieurs prébiotiques choisis parmi les compositions d'oligosaccharides non digestibles comprenant des enchaînements d'oses identiques ou différents, et dont le degré de polymérisation varie entre 2 et 10, et de préférence entre 3 et 8, pour son utilisation dans l'alimentation des sujet risquant de développer un syndrome hyperglycémique, dans le cadre de la prévention des syndromes hyperglycémiques, et dans l'alimentation des sujets atteints d'obésité, ou susceptibles de développer une obésité, et présentant une prédisposition à développer un diabète de type II, à savoir chez les sujets présentant des signes cliniques prédictifs de ce diabète, tels qu'une diminution de la tolérance au glucose, ou de la sensibilité à l'insuline, chez les sujets présentant une prédisposition héréditaire à développer ce type de diabète, ou liée à leurs habitudes alimentaires, dans le cadre de la prévention de l'apparition d'un diabète de type II chez ces sujets, lesdits oligosaccharides étant choisis parmi les gluco-oligosaccharides (GOS), à savoir des polymères de glucose de formule générale [O-α-D-glucopyranosyl]ₙ où n est un nombre entier de 2 à 10, et de préférence de 3 à 8, tels que les polymères de formule [O-α-D-glucopyranosyl-(1→2)][O-α-D-glucopyranosyl-(1→6)]ₙ[O-α-D-glucopyranosyl-(1→4)]O-D-glucopyranose où n est un nombre entier de 1 à 10, et la position de la liaison α(1→2) se situe soit à l'extrémité non réductrice, soit se situe en ramification sur l'avant dernier glucose de la chaîne, ou les polymères de type malto-oligosaccharides de formule générale [O-α-D-glucopyranosyl-(1 →4)]ₙ où n est un nombre entier de 2 à 10, ou les isomalto-oligosaccharides de formule générale [O-α-D-glucopyranosyl-(1→6)]ₙ où n est un nombre entier de 2 à 10,
la teneur en matière sèche de la composition desdits gluco-oligosaccharides (GOS) étant la suivante :
- fructose : moins de 1%,
- glucose : moins de 4%,
- disaccharides (maltose, leucrose, sacharose) : de 9 à 11%,
- trisaccharides (panose, maltotriose) : de 9 à 11%,
- GOS de degré de polymérisation 4 : de 5 à 7%,
- GOS* de degré de polymérisation 4 : de 8 à 10%,
- GOS de degré de polymérisation 5 : de 18 à 22%,
- GOS de degré de polymérisation supérieur à 5 : de 36 à 44%,
chaque GOS comportant une liaison glycosidique α(1 → 2) à son extrémité non-réductrice ou portée par l'avant dernier glucose, excepté le GOS* marqué par un astérisque qui n'en contient pas.

4. Composition pharmaceutique **caractérisée en ce qu'**elle comprend un ou plusieurs prébiotiques en association avec un véhicule pharmaceutiquement acceptable, et **en ce qu'**elle comprend un ou plusieurs prébiotiques choisis parmi les compositions d'oligosaccharides non digestibles comprenant des enchaînements d'oses identiques ou différents, et dont le degré de polymérisation varie entre 2 et 10, et de préférence entre 3 et 8, lesdits oligosaccharides étant choisis parmi les gluco-oligosaccharides (GOS), à savoir des polymères de glucose de formule générale [O-α-D-glucopyranosyl]ₙ où n est un nombre entier de 2 à 10, et de préférence de 3 à 8, tels que les polymères de formule générale [O-α-D-glucopyranosyl-(1 → 2)][O-α-D-glucopyranosyl-(1 → 6)]ₙ[[O-α-D-glucopyranosyl-(1 → 4)]O-D-glucopyranose où n est un nombre entier de 1 à 10, et la position de la liaison a(1 → 2) se situe soit à l'extrémité non réductrice, soit se situe en ramification sur l'avant dernier glucose de la chaîne, ou les polymères de type malto-oligosaccharides de formule générale [O-α-D-glucopyranosyl-(1 → 4)]ₙ où n est un nombre entier de 2 à 10, ou les isomalto-oligosaccharides de formule générale [O-α-D-glucopyranosyl-(1 → 6)]ₙ où n est un nombre entier de 2 à 10,
la teneur en matière sèche de la composition desdits gluco-oligosaccharides (GOS) étant la suivante :
- fructose ; moins de 1%,
- glucose : moins de 4%,
- disaccharides (maltose, leucose, sacharose) : de 9 à 11%,
- trisaccharides (panose, maltotriose) : de 9 à 11%,
- GOS de degré de polymérisation 4 : de 5 à 7%,
- GOS* de degré de polymérisation 4 : de 8 à 10%,
- GOS de degré de polymérisation 5 : de 18 à 22%,
- GOS de degré de polymérisation supérieur à 5 : de 36 à 44%,
chaque GOS comportant une liaison glycosidique α(1 → 2) à son extrémité non-réductrice ou portée par l'avant dernier glucose, excepté le GOS* marqué par un astérisque qui n'en contient pas.

5. Composition pharmaceutique selon la revendication 3-4 **caractérisée en ce qu'**elle se présente sous une forme administrable par voie orale.

6. Composition pharmaceutique selon l'une des revendications 3-5, **caractérisée en ce qu'**elle est administrée à raison d'environ 10 à 30 g/jour, jusqu'à environ 100 g/jour dans le cas de l'utilisation de GOS.

## Claims

1. Use of prebiotics for the preparation of food or pharmaceutical compositions intended for the prevention of hyperglycemic syndromes and for the prevention of the appearance of a type II diabetes in subjects presenting a predisposition to develop this type of diabetes, said prebiotics being chosen from the compositions of non-digestible oligosaccharides comprising the succession of identical or different monosaccharides, and whose degree of polymerization varies between 2 and 10, and preferably between 3 and 8, said oligonucleotides being chosen from glucooligosaccharides (GOS), namely glucose polymers of general formula [O-α-D-glucopyranosyl]ₙ where n is an integer from 2 to 10, and preferably from 3 to 8, such as the polymers of general formula [O-α-D-glucopyranosyl-(1 → 2)][O-α-D-glucopyranosyl-(1 → 6)]ₙ[O-α-D-glucopyranosyl-(1 → 4)]O-D-glucopyranose where n is an integer from 1 to 10, and the position of the α(1 → 2) bond is situated either at the non-reducing end, or is situated in a branched position on the next-to-last glucose of the chain, or the polymers of the maltooligosaccharide type of general formula [O-α-D-glucopyranosyl-(1 → 4)]ₙ where n is an integer from 2 to 10, or isomaltooligosaccharides of general formula [O-α-D-glucopyranosyl-(1 → 6)]ₙ where n is an integer from 2 to 10,
the dry matter content of the composition of glucooligosaccharides (GOS) being as follows:
- fructose: less than 1%,
- glucose: less than 4%,
- disaccharides (maltose, leucrose, sacharose): from 9 to 11%,
- trisaccharides (panose, maltotriose): from 9 to 11 %,
- GOS with a degree of polymerization 4: from 5 to 7%,
- GOS* with a degree of polymerization 4: from 8 to 10%,
- GOS with a degree of polymerization 5: from 18 to 22%,
- GOS with a degree of polymerization greater than 5: from 36 to 44%,
each GOS comprising a glycosidic α(1 → 2) bond at its non-reducing end or carried by the next-to-last glucose, except for the GOS* marked by an asterisk which does not contain any.

2. Use of prebiotics according to claim 1 for the preparation of food or pharmaceutical compositions intended for the prevention of hyperglycemic syndromes and for the prevention of the appearance of a type II diabetes in subjects presenting a predisposition to develop this type of diabetes, at a rate of approximately 10 to 30 g/day, up to approximately 100 g/day in the case of the use of GOS.

3. Food composition, nutritional additive, functional food or nutraceutical, comprising one or more prebiotics chosen from the compositions of non-digestible oligosaccharides comprising the succession of identical or different monosaccharides, and whose degree of polymerization varies between 2 and 10, and preferably between 3 and 8, for its use in the nourishment of subjects at risk of developing an hyperglycemic syndrome, in the context of the prevention of hyperglycemic syndromes, and in the nourishment of subjects suffering from obesity, or at risk of becoming obese, and presenting a predisposition to develop a type II diabetes, namely in subjects presenting predictive clinical signs of this diabetes, such as a decrease in glucose tolerance, or sensitivity to insulin, in subjects presenting a hereditary predisposition to develop this type of diabetes, or linked to their eating habits, in the context of-preventing the appearance of a type II diabetes in these subjects, said oligonucleotides being chosen from glucooligosaccharides (GOS), namely glucose polymers of general formula [O-α-D-glucopyranosyl]ₙ where n is an integer from 2 to 10, and preferably from 3 to 8, such as the polymers of general formula [O-α-D-glucopyranosyl-(1 → 2)][O-α-D-glucopyranosyl-(1 → 6)]ₙ[O-α-D-glucopyranosyl-(1 → 4)]O-D-glucopyranose where n is an integer from 1 to 10, and the position of the α(1 → 2) bond is situated either at the non-reducing end, or is situated in a branched position on the next-to-last glucose of the chain, or the polymers of the maltooligosaccharide type of general formula [O-α-D-glucopyranosyl-(1 → 4)]n where n is an integer from 2 to 10, or isomaltooligosaccharides of general formula [O-α-D-glucopyranosyl-(1 → 6)]ₙ where n is an integer from 2 to 10, the dry matter content of the composition of glucooligosaccharides (GOS) being as follows:
- fructose: less than 1%,
- glucose: less than 4%,
- disaccharides (maltose, leucrose, sacharose): from 9 to 11 %,
- trisaccharides (panose, maltotriose): from 9 to 11%,
- GOS with a degree of polymerization 4: from 5 to 7%,
- GOS* with a degree of polymerization 4: from 8 to 10%,
- GOS with a degree of polymerization 5: from 18 to 12%,
- GOS with a degree of polymerization greater than 5: from 36 to 44%,
each GOS comprising a glycosidic α(1 → 2) bond at its non-reducing end or carried by the next-to-last glucose, except for the GOS* marked by an asterisk which does not contain any.

4. Pharmaceutical composition **characterized in that** it comprises one or more prebiotics in combination with a pharmaceutically acceptable vehicle, and **in that** comprises one or more prebiotics chosen from the compositions of non-digestible oligosaccharides comprising the succession of identical or different monosaccharides, and whose degree of polymerization varies between 2 and 10, and preferably between 3 and 8, said oligonucleotides being chosen from glucooligosaccharides (GOS), namely glucose polymers of general formula [O-α-D-glucopyranosyl]ₙ where n is an integer from 2 to 10, and preferably from 3 to 8, such as the polymers of general formula [O-α-D-glucopyranosyl-(1 → 2)][O-α-D-glucopyranosyl-(1 → 6)]ₙ[O-α-D-glucopyranosyl-(1 → 4)]O-D-glucopyranose where n is an integer from 1 to 10, and the position of the α(1 → 2) bond is situated either at the non-reducing end, or is situated in a branched position on the next-to-last glucose of the chain, or the polymers of the maltooligosaccharide type of general formula [O-α-D-glucopyranosyl-(1 → 4)]ₙ where n is an integer from 2 to 10, or isomaltooligosaccharides of general formula [O-α-D-glucopyranosyl-(1 → 6)]ₙ where n is an integer from 2 to 10,
the dry matter content of the composition of glucooligosaccharides (GOS) being as follows:
- fructose: less than 1%,
- glucose: less than 4%,
- disaccharides (maltose, leucrose, sacharose): from 9 to 11 %,
- trisaccharides (panose, maltotriose): from 9 to 11%,
- GOS with a degree of polymerization 4: from 5 to 7%,
- GOS* with a degree of polymerization 4: from 8 to 10%,
- GOS with a degree of polymerization 5: from 18 to 22%.
- GOS with a degree of polymerization greater than 5: from 36 to 44%,
each GOS comprising a glycosidic α(1 → 2) bond at its non-reducing end or carried by the next-to-last glucose, except for the GOS* marked by an asterisk which does not contain any.

5. Pharmaceutical composition according to claim 3-4, **characterized in that** it is in a form which can be administered by oral route.

6. Pharmaceutical composition according to one of claims 3-5, **characterized in that** it is administered at a rate of approximately 10 to 30 g/day, up to approximately 100 g/day in the case of the use of GOS.

## Patentansprüche

1. Verwendung von Präbiotika zur Herstellung von Nahrungsmittelzusammensetzungen oder pharmazeutischen Zusammensetzungen, d ie zur V orbeugung von hyperglykämischen Syndromen und zur Vorbeugung des Auftretens eines Typ-II-Diabetes bei Subjekten bestimmt sind, die eine Prädisposition zur Entwicklung dieses Diabetestyps aufweisen, wobei die Präbiotika aus den unverdaulichen Oligosaccharidzusammensetzungen ausgewählt sind, die Ketten von gleichen oder verschiedenen Monosacchariden umfassen und deren Polymerisationsgrad zwischen 2 und 10 und bevorzugt zwischen 3 und 8 variiert, wobei die Oligosaccharide aus den Gluco-Oligosacchariden (GOS) ausgewählt sind, nämlich den Glucosepolymeren mit der allgemeinen Formel [O-α-D-Glucopyranosyl]ₙ, worin n eine ganze Zahl von 2 bis 10 und bevorzugt von 3 bis 8 ist, wie etwa den Polymeren mit der allgemeinen Formel [O-α-D-Glucopyranosyl-(1→2)] [O-α-D-glucopyranosyl-(1→6)]ₙ[O-α-D-glucopyranosyl-(1→4)]O-D-glucopyranose, worin n eine ganze Zahl von 1 bis 10 ist, und die Position der Bindung α(1→2) entweder am nichtreduzierenden Ende liegt oder an der Verzweigung auf der vorletzten Glucose der Kette liegt, oder den Polymeren vom Typ der Malto-Oligosaccharide mit der allgemeinen Formel [O-α-D-Glucopyranosyl-(1→4)]ₙ, worin n eine ganze Zahl von 2 bis 10 ist, oder den Isomalto-Oligosacchariden mit der allgemeinen Formel [O-α-D-Glucopyranosyl-(1→6)]ₙ, worin n eine ganze Zahl von 2 bis 10 ist,
wobei der Trockengehalt der Zusammensetzung der Gluco-Oligosaccharide (GOS) Folgender ist:
- Fructose: weniger als 1 %,
- Glucose: weniger als 4 %,
- Disaccharide (Maltose, Leucrose, Saccharose): 9 bis 11 %,
- Trisaccharide (Panose, Maltotriose): 9 bis 11 %,
- GOS mit Polymerisationsgrad 4: 5 bis 7 %,
- GOS* mit Polymerisationsgrad 4: 8 bis 10 %,
- GOS mit Polymerisationsgrad 5: 18 bis 22 %,
- GOS* mit einem Polymerisationsgrad größer als 5: 36 bis 44 %,
wobei jedes GOS an seinem nichtreduzierenden Ende eine Glycosidbindung α(1→2) umfasst oder diese von der vorletzten Glucose getragen wird, abgesehen von GOS*, das mit einem Stern markiert ist, das keine enthält.

2. Verwendung von Präbiotika nach Anspruch 1 zur Herstellung von Nahrungsmittelzusammensetzungen oder pharmazeutischen Zusammensetzungen, die zur Vorbeugung von hyperglykamischen Syndromen und zur Vorbeugung des Auftretens eines Typ-II-Diabetes bei Subjekten bestimmt sind, die eine Prädisposition zur Entwicklung dieses Diabetestyps aufweisen, in einer Menge von etwa 10 bis 30 g/Tag bis etwa 100 g/Tag.

3. Nahrungsmittelzusammensetzung, Nährzusatzstoff, funktionelles Lebensmittel oder Nutrazeutikum, umfassend ein oder mehrere Präbiotika, die aus den unverdaulichen Oligosaccharidzusammensetzungen ausgewählt sind, die Ketten von gleichen oder verschiedenen Monosacchariden umfassen und deren Polymerisationsgrad zwischen 2 und 10 und bevorzugt zwischen 3 und 8 variiert, für deren/dessen Verwendung bei der Ernährung von Subjekten, bei denen die Gefahr der Entwicklung eines hyperglykämischen Syndroms besteht, im Rahmen der Vorbeugung von hyperglykämischen Syndromen und bei der Ernährung von Subjekten, die an Fettleibigkeit leiden oder die für die Entwicklung einer Fettleibigkeit anfällig sind, und die eine Prädisposition zur Entwicklung eines Typ-II-Diabetes aufweisen, nämlich bei Subjekten, die klinische Vorzeichen dieses Diabetes aufweisen, wie etwa eine Verringerung der Glucosetoleranz oder eine Insulinsensitivität bei Subjekten, die eine erbliche Prädisposition zur Entwicklung dieses Diabetestyp aufweisen, oder die mit ihren Ernährungsgewohnheiten verbunden ist, im Rahmen der Vorbeugung des Auftretens eines Typ-II-Diabetes bei diesen Subjekten, wobei die Oligosaccharide aus den Gluco-Oligosacchariden (GOS) ausgewählt sind, nämlich den Glucosepolymeren mit der allgemeinen Formel .[O-α-D-Glucopyranosyl]ₙ, worin n eine ganze Zahl von 2 bis 10 und bevorzugt von 3 bis 8 ist, wie etwa den Polymeren mit der allgemeinen Formel [O-α-D-Glucopyranosyl-(1→2)][O-α-D-glucopyranosyl-(1→6)]ₙ[O-α-D-glucopyranosyl-(1→4)]O-D-glucopyranose, worin n eine ganze Zahl von 1 bis 10 ist, und die Position der Bindung α(1→2) entweder am nichtreduzierenden Ende liegt oder an der Verzweigung an der vorletzten Glucose der Kette liegt, oder den Polymeren vom Typ der Malto-Oligosaccharide mit der allgemeinen Formel [O-α-D-Glucopyranosyl-(1→4)]ₙ, worin n eine ganze Zahl von 2 bis 10 ist, oder den Isomalto-Oligosacchariden mit der allgemeinen Formel [O-α-D-Glucopyranosyl-(1→6)]ₙ, worin n eine ganze Zahl von 2 bis 10 ist,
wobei der Trockengehalt der Zusammensetzung der Gluco-Oligosaccharide (GOS) Folgender ist:
- Fructose: weniger als 1 %,
- Glucose: weniger als 4 %,
- Disaccharide (Maltose, Leucrose, Saccharose): 9 bis 11 %,
- Trisaccharide (Panose, Maltotriose): 9 bis 11 %,
- GOS mit Polymerisationsgrad 4: 5 bis 7 %,
- GOS* mit Polymerisationsgrad 4: 8 bis 10 %,
- GOS mit Polymerisationsgrad 5: 18 bis 22 %,
- GOS* mit einem Polymerisationsgrad größer als 5: 36 bis 44 %,
wobei jedes GOS an seinem nichtreduzierenden Ende eine Glycosidbindung α(1→2) umfasst oder diese von der vorletzten Glucose getragen wird, abgesehen von GOS*, das mit einem Stern markiert ist, das keine enthält.

4. Pharmazeutische Zusammensetzung, **dadurch gekennzeichnet, dass** sie ein oder mehrere Präbiotika in Assoziation mit einem pharmazeutisch verträglichen Vehikel umfasst, und dadurch, dass sie ein oder mehrere Präbiotika umfasst, die aus den unverdaulichen Oligosacchariden ausgewählt sind, die Ketten von gleichen oder verschiedenen Monosacchariden umfassen und deren Polymerisationsgrad zwischen 2 und 10 und bevorzugt zwischen 3 und 8 variiert, wobei die Oligosaccharide aus den Gluco-Oligosacchariden (GOS) ausgewählt sind, nämlich den Glucosepolymeren mit der allgemeinen Formel [O-α-D-Glucopyranosyl]ₙ, worin n eine ganze Zahl von 2 bis 10 und bevorzugt von 3 bis 8 ist, wie etwa den Polymeren mit der allgemeinen Formel [O-α-D-Glucopyranosyl-(1→2)][O-α-D-glucopyranosyl-(1→6)]ₙ[O-α-D-glucopyranosyl-(1→4)]O-D-glucopyranose, worin n eine ganze Zahl von 1 bis 10 ist, und die Position der Bindung α(1→2) entweder am nichtreduzierenden Ende liegt oder an der Verzweigung auf der vorletzten Glucose der Kette liegt, oder den Polymeren vom Typ der Malto-Oligosaccharide mit der allgemeinen Formel [O-α-D-Glucopyranosyl-(1→4)]ₙ, worin n eine ganze Zahl von 2 bis 10 ist, oder den Isomalto-Oligosacchariden mit der allgemeinen Formel [O-α-D-Glucopyranosyl-(1→6)]ₙ, worin n eine ganze Zahl von 2 bis 10 ist,
wobei der Trockengehalt der Zusammensetzung der Gluco-Oligosaccharide (GOS) Folgender ist:
- Fructose: weniger als 1 %,
- Glucose: weniger als 4 %,
- Disaccharide (Maltose, Leucrose, Saccharose): 9 bis 11 %,
- Trisaccharide (Panose, Maltotriose): 9 bis 11 %,
- GOS mit Polymerisationsgrad 4: 5 bis 7 %,
- GOS* mit Polymerisationsgrad 4: 8 bis 10 %,
- GOS mit Polymerisationsgrad 5: 18 bis 22 %,
- GOS* mit einem Polymerisationsgrad größer als 5: 36 bis 44%,
wobei jedes GOS an seinem nichtreduzierenden Ende eine Glycosidbindung α(1→2) umfasst oder diese von der vorletzten Glucose getragen wird, abgesehen von GOS*, das mit einem Stern markiert ist, das keine enthält.

5. Pharmazeutische Zusammensetzung nach Anspruch 3 bis 4, **dadurch gekennzeichnet, dass** sie eine oral verabreichbare Form aufweist.

6. Pharmazeutische Zusammensetzung nach einem der Ansprüche 3 bis 5, **dadurch gekennzeichnet, dass** sie in einer Menge von etwa 10 bis 30 g/Tag bis etwa 100 g/Tag, im Fall der Verwendung von GOS, verabreicht wird.
